# DEMANDE DE BREVET EUROPEEN

(11) **EP 0 716 837 A2**
(43) Date de publication de la demande: **19.06.1996**
(21) Numéro de dépôt: 95402767.8
(22) Date de dépôt: 08.12.1995
(51) Int. Cl.: A61F 2/26

(54) **Implants d'extension caverneuse**

(30) Priorité: 08.12.1994 FR 9414758
(71) Demandeur: Subrini, Louis, Dr., F-78370 Plaisir (FR)
(72) Inventeur: Subrini, Louis, Dr., F-78370 Plaisir (FR)

(57) **Abrégé**

L'invention concerne un implant d'extension caverneuse permettant d'allonger les corps caverneux de façon irréversible et permanente, par utilisation du phénomène d'expansion tissulaire.

II est constitué d'une chambre de pression 2 pourvue d'une valve 12, et d'une une gaine 3, dans laquelle coulisse le corps de l'implant 7. Après section à la longueur voulue, son extrémité est coiffée d'un embout 10 creusé d'une gorge 9 et prolongé d'une collerette 8. L'adjonction de fluide par ponction de la chambre 2 par l'intermédiaire d'un détendeur de pression 11 repousse le corps de l'implant, allongeant ainsi l'ensemble du dispositif. Des injections répétitives permettent l'allongement progressif des corps caverneux jusqu'à obtenir la longueur de verge désirée. Le dispositif peut alors être enlevé.

Le dispositif selon l'invention est destiné à allonger la verge.

## Description

La présente invention concerne un implant d'extension caverneuse comprenant un dispositif d'extension irréversible, dont le but est d'allonger les corps caverneux de façon importante et définitive, par expansion tissulaire.

L'expansion tissulaire est un phénomène physiologique selon lequel tout tissu vivant soumis à une tension, s'allonge jusqu'à ce que la tension soit nulle. Le phénomène est reproductible, toute nouvelle tension entraînant un nouvel allongement. Dans la présente invention le but étant d'allonger la verge, on recherchera une expansion tissulaire des corps caverneux dans le sens longitudinal.

L'inconvénient des implants péniens existants même ceux qui peuvent s'allonger par un mécanisme quelconque, est qu'ils sont incapables d'allonger les corps caverneux. Ils ne sont d'ailleurs pas conçus pour cette usage, mais destinés uniquement à permettre la pénétration à des sujets impuissants, en donnant à la verge une rigidité artificielle.

En effet, es implants péniens existants qu'ils soient inertes ou gonflables, ont tous au repos une longueur constante. Le souci d'obtenir une verge plus longue et surtout plus rigide à l'état d'érection qu'à l'état de repos a conduit à concevoir des prothèses dont la longueur peut s'adapter à l'aide d'un soufflet ou d'un système analogue. A l'état de flaccidité, les prothèses sont à leur longueur minimum. Lorsque le patient désire une érection, il active un système de pompe, ce qui entraîne une augmentation de la rigidité des prothèses, puis un certain degré d'allongement du moins jusqu'à ce qu'elles occupent toute la longueur disponible du corps caverneux. Après les rapports sexuels, l'opéré désactive le système, et les prothèses perdent alors leur rigidité et retrouvent leur longueur initiale de repos.

L'inconvénient de tels systèmes est que la différence de longueur des prothèses entre les deux états de flaccidité et de rigidité est limitée, de l'ordre de 2 centimètres environ. Un autre inconvénient est que cette différence de longueur des prothèses est réversible et temporaire, ne se produit qu'à l'état d'érection artificielle, et est donc incapable d'induire une expansion tissulaire des corps caverneux. L'allongement des prothèses entraîne en revanche leur raidissement ce qui est le but recherché.

D'autres prothèses péniennes d'érection comportent un dispositif de réglage de leur longueur, de façon à adapter de façon aussi précise que possible la longueur de la prothèse à celle du corps caverneux. Le raccourcissement ou l'allongement de la prothèse est obtenu par injection ou par retrait d'un fluide d'un soufflet, selon que la prothèse est trop longue ou trop courte pour le corps caverneux où elle est insérée. Là encore le dispositif ne peut s'allonger que dans la mesure où la longueur du corps caverneux le permet, et en tout cas de façon négligeable.

De façon générale les inconvénients de ces systèmes sont qu'ils sont conçus pour rigidifier la verge lors de la pénétration par le biais d'un allongement limité et réversible. Ils ne procurent aucun allongement des corps caverneux, fonction pour laquelle ils ne sont d'ailleurs pas conçus.

Un autre inconvénient de ces dispositifs tient au fait que l'allongement des prothèses fait habituellement appel à un soufflet, ou plus précisément à des ondulations dont l'épaisseur est constante et qui lui donnent une forme annelée. Or toute enceinte déformable dont la paroi est d'une épaisseur constante et dont la pression interne augmente, tend à prendre une forme sphérique. Ainsi une telle enceinte qui comporte des plis repliés sur eux-mêmes en forme de soufflet, lorsqu'elle est soumise à une augmentation de pression, aura tendance non seulement à s'allonger par dépliage du soufflet, mais encore à grossir par augmentation de son diamètre transversal. Une partie de l'énergie est donc utilisée à augmenter le diamètre au lieu d'être entièrement disponible pour entraîner une extension du système dans une direction préférentielle, c'est à dire dans le sens de l'allongement, qui est le but recherché par la présente invention.

Un autre inconvénient de la forme annelée est que chaque pli est de faible hauteur, si bien que l'allongement du système même entièrement déplié reste faible, sauf à multiplier le nombre des plis, ce que la longueur du corps caverneux permet d'autant moins que la verge est courte.

Or, certains patients impuissants ou non présentent une verge courte, qui nécessite d'allonger les corps caverneux non pas de façon temporaire lors de l'érection, mais au contraire de façon définitive et importante, afin de donner à la verge une longueur normale, ce que la présente invention se propose de réaliser.

Son but est d'allonger les corps caverneux grâce à un implant pourvu d'un dispositif d'extension, qui grâce à une source extérieure de fluide peut s'allonger graduellement jusqu'à 6 à 10 centimètres, sans augmentation obligatoire de son diamètre transversal.

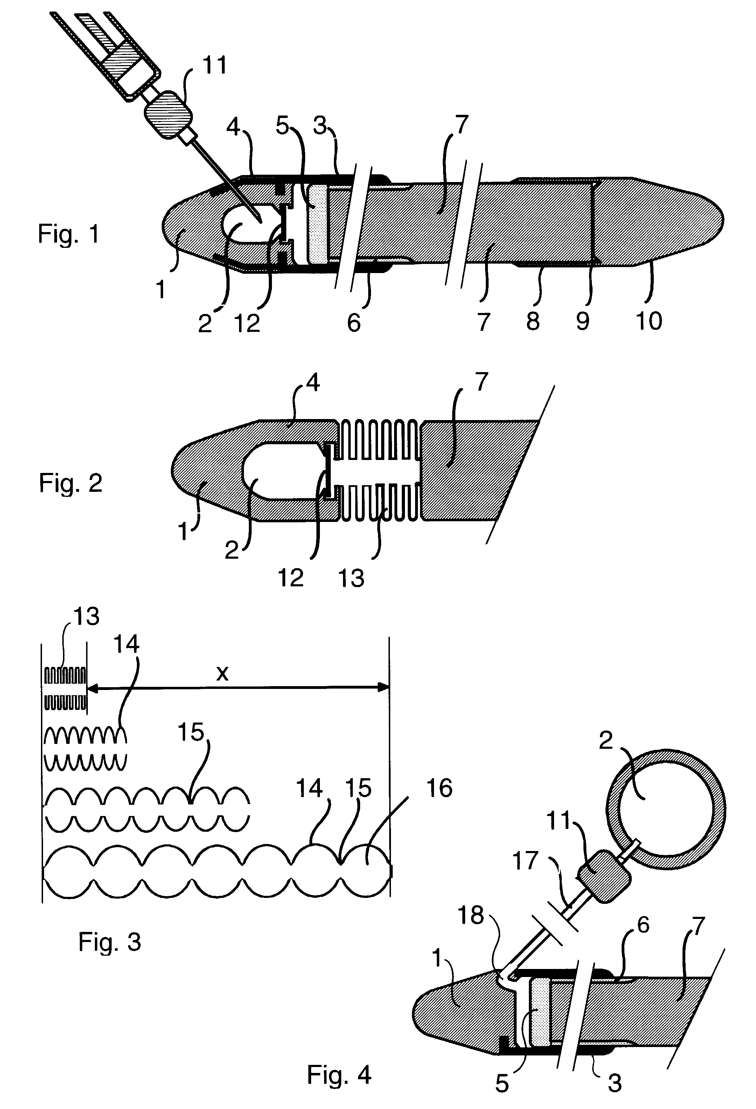

L'implant pénien selon une première caractéristique, se présente sous une forme allongée, de préférence de section circulaire tel que représenté dans la figure 1 qui forme un ensemble monobloc. Cet ensemble comprend : le corps de l'implant 7, une chambre de pression de forme extérieure sphéro-conique, creusée d'une cavité qui constitue un site d'injection, et qui communique par l'intermédiaire d'une valve anti-retour avec le dispositif d'extension. Le corps de l'implant 7 est constitué d'un matériau synthétique plein et de préférence homogène dont la dureté est comprise entre 20 et 50 shore A environ. Il est destiné à être coupé sur mesure à la longueur désirée, puis éventuellement coiffé d'un embout 10 dont l'extrémité est identique ou semblable à celle de l'extrémité opposée. Lors de sa mise en place, chaque ensemble est d'une longueur telle qu'il occupe toute la longueur disponible du corps caverneux où il est inséré. Chacune des extrémités de l'ensemble vient se loger dans le cul-de-sac correspondant de chaque corps caverneux au fond duquel elle s'appuie.

Une chambre de pression qui constitue un site d'injection de fluide, communique par l'intermédiaire d'une valve avec un dispositif d'extension constitué d'une gaine dans laquelle coulisse le corps de l'implant. L'adjonction de fluide par une injection grâce à une aiguille fine dans la chambre augmente sa pression interne, repousse le corps de l'implant et allonge l'ensemble, ce qui provoque une expansion tissulaire longitudinale du corps caverneux. La vitesse de l'expansion tissulaire est d'autant plus grande que la pression d'injection et le volume injecté sont importants. Dès que l'allongement du corps caverneux a atteint un certain degré, l'injection doit être renouvelée, induisant ainsi une nouvelle expansion tissulaire caverneuse, et ainsi de suite jusqu'à obtenir la longueur de verge désirée. La pression dans une seringue pouvant être très élevée, un détendeur qui réalise un système simple de régulateur de pression ne permet l'adjonction de fluide dans la chambre, qu'à une pression inférieure à un seuil pré-déterminé et réglable, afin d'éviter toute hyperpression non désirée.

Selon des modalités particulières de réalisation,
- le segment extensible du dispositif peut être en forme d'accordéon, composé par un empilement de plusieurs cylindres de faible hauteur, réalisant autant de disques creux communiquant les uns avec les autres.
- le segment extensible du dispositif peut être constitué par un système de ressort mis en place sous sa forme comprimée, qui allonge progressivement l'implant sous l'effet de sa propre énergie.
- L'adjonction de fluide peut se faire grâce à un système de pompe annexé au dispositif.
- La chambre de pression, le détendeur, et la pompe peuvent être isolément ou en totalité être situés en dehors des corps cavemeux et séparés du corps de l'implant. Ils lui sont alors reliés par une ou des tubulures appropriées, réalisant ainsi un ensemble bi-corps ou multi-corps.

Les dessins annexés illustrent l'invention.

La figure 1 représente une coupe du dispositif selon l'invention où le segment extensible est constitué par le corps de l'implant coulissant dans une gaine.

La figure 2 représente une variante du dispositif, où le segment extensible est constitué d'un empilement de disques creux.

La figure 3 illustre le résultat obtenu par l'augmentation de la pression interne dans les disques creux.

La figure 4 représente en coupe une variante du dispositif, où la chambre de pression et le détendeur de pression sont distincts du corps de l'ensemble.

En référence à ces dessins, l'implant pénien d'extension selon une première caractéristique, se présente sous la forme d'un corps de section circulaire ou elliptique, tel que représenté dans la figure 1 où le dispositif d'extension est intégré dans un ensemble monobloc. L'extrémité 1 est de forme arrondie ou sphéro-conique, de façon à être bien adaptée à l'anatomie interne du cul-de-sac cavemeux. L'extrémité opposée du dispositif est destinée à être coupée sur mesure à la longueur désirée, puis coiffée d'un embout 10 de forme identique ou semblable à celle de l'extrémité 1.

Le dispositif d'extension se compose d'une chambre de pression 2 et d'une une gaine 3, dans laquelle coulisse le corps de l'implant 7. Les parois de la chambre de pression sont constituées d'un matériau bio-compatible qui peut présenter si nécessaire un renforcement 4 par exemple en Dacron, de façon à résister à l'augmentation de pression et à permettre plusieurs injections de fluide dans la chambre, sans pour autant entraîner de fuite de liquide par le trou laissé par l'aiguille. Selon une des modalités de l'invention, la chambre de pression 2 peut être pourvue d'une valve 12 résistante, par exemple métallique qui rend irréversible tout passage de fluide de la chambre vers le segment extensible. Ainsi même au cas de fuite à travers la paroi de la chambre de pression, le segment extensible du dispositif conserve sa pression interne. De plus, la valve réalise une butée empêchant l'aiguille de pénétrer dans le dispositif extensible.

La gaine 3 est de faible épaisseur, de manière à ce que son diamètre extérieur soit le plus proche possible de celui du corps de l'implant, afin de conserver à l'ensemble un diamètre extérieur quasi constant. La gaine présente une surface intérieure adaptée au corps de l'implant de façon à assurer l'étanchéité du système, et est d'une longueur autorisant une course du corps de l'implant de l'ordre de 8 centimètres environ.

Le corps de l'implant 7 est chargé de transmettre la pression interne du dispositif d'extension au corps caverneux dans lequel il est implanté, afin d'entraîner son allongement. Le corps de l'implant est de préférence de section cylindrique. Son extrémité peut s'appuyer sur un piston 5, ou être en partie engainé dans un cylindre creux 6. Le corps de l'implant peut être solidarisé au piston lors de la fabrication, par exemple par collage, à moins que le chirurgien ne l'insère lui-même dans le cylindre creux, avant la mise en place du dispositif dans le corps caverneux. En pareil cas il lui est loisible si nécessaire, de raccourcir le corps de l'implant avant son introduction dans le cylindre 6, par section transversale au bistouri ou aux ciseaux. Le corps de l'implant est constitué de silicone ou de toute autre matériau bien toléré par l'organisme. Selon une des modalités de l'invention, ce matériau peut présenter une faible résistance à la compression axiale, ce qui permet au corps de l'implant d'amortir les effets d'un éventuel excès de pression non souhaité. Selon une des modalités de l'invention, à titre d'exemple non limitatif, un tel système amorti est assuré par exemple par un élastomère de silicone, qui sous l'effet d'une compression axiale de 500 grammes subit une déformation qui raccourcit un corps d'implant cylindrique de 12 mm de diamètre, de l'ordre de 2 à 6 %, soit pour une longueur de 15 cm un raccourcissement de l'ordre de 6 mm. Une telle fonction peut être assurée par exemple par un élastomère de silicone d'une dureté de l'ordre de 20 à 50 shore A environ.

L'embout 10 est de forme arrondie, de préférence sphéro-conique et se prolonge par une collerette 8. Il peut être constitué d'un matériau analogue ou plus souple que celui du corps de l'implant. Après que le corps de l'implant 7 ait été coupé à la longueur voulue, il est coiffé de l'embout dont la forme est bien adaptée au cul-de-sac caverneux qui doit le recevoir. L'embout peut avantageusement être creusé d'une ou plusieurs cavités, par exemple d'une gorge périphérique 9 de manière à ce que l'on peut en repoussant la collerette sur l'implant, vider tout ou partie de la cavité et entraîner ainsi un effet de ventouse, apte à résister à l'arrachement de l'embout.

Lors de sa mise en place, l'ensemble de l'implant d'extension cavemeuse est d'une longueur totale telle qu'il occupe toute la longueur disponible du corps cavemeux où il est inséré. Chacune des extrémités de l'ensemble venant se loger dans le cul-de-sac correspondant de chaque corps caverneux au fond duquel elle s'appuie.

La ponction directe de la chambre 2 effectuée à travers la peau et la paroi du corps caverneux, par une aiguille fine reliée à une seringue, permet l'injection de fluide, par exemple de sérum physiologique dans la chambre, dont la pression interne augmente, et repousse le corps de l'implant allongeant ainsi l'ensemble du dispositif.

Un détendeur de pression 11 peut être intercalé entre la seringue et l'aiguille, afin de ne délivrer de fluide, qu'à une pression inférieure à un seuil prédéterminé ou réglable. On évite ainsi toute hyperpression non désirée dans le dispositif. La pression sur les deux extrémités opposées des corps caverneux provoque une expansion tissulaire longitudinale du corps caverneux qui s'allonge progressivement. Selon une des modalités de l'invention, dès que l'allongement du corps caverneux a atteint un certain degré, l'injection est renouvelée, induisant ainsi une nouvelle expansion tissulaire caverneuse, et ainsi de suite jusqu'à obtenir la longueur de verge désirée. La fréquence et le volume des injections est évidemment variable selon la pathologie en cause, la rapidité et l'importance de l'allongement désiré. Elle peut être lente, par exemple au rythme d'une injection de 1 millilitre tous les 20 jours, ou au contraire rapide avec une seule injection continue pendant une durée de l'ordre de l'heure, pour autant que le permettent le réglage du détendeur de pression et la souplesse du corps caverneux.

Les implants péniens d'extension sont insérés soit de façon définitive, soit de façon temporaire. Dans ce dernier cas, ils sont enlevés lorsque l'allongement souhaité a été obtenu, et peuvent éventuellement être remplacés par des implants péniens définitifs.

Selon une autre modalité de l'invention, le système s'allonge par l'utilisation de sa propre énergie. Un tel dispositif peut être constitué dans l'une de ses applications par exemple par un système de ressort non représenté, qui est mis en place sous sa forme comprimée au contact de l'extrémité du corps de l'implant, et qui en se détendant, allonge progressivement le dispositif.

Selon une des modalités de l'invention, le segment extensible est composé d'un empilement de plusieurs cylindres de faible hauteur, réalisant autant de disques creux 13, l'ensemble se présentant sous la forme d'une sorte accordéon, tel qu'illustré par la figure 2. Les cavités des disques communiquent entre elles au niveau de l'axe des disques, et sont emplies d'un fluide, par exemple de sérum physiologique. Chacun des disques creux 13 constituant du dispositif, se présente initialement c'est à dire avant l'insertion de l'implant dans le corps cavemeux, de façon à ce que chaque face soit accolée aux faces adjacentes, et n'en soit séparée que par un espace virtuel ou quasi virtuel. Ce n'est que pour la lisibilité des figures, que les disques y sont représentés séparés par un espace. La paroi de chaque disque est constituée d'un matériau bien toléré par l'organisme, comme le silicone par exemple, dont la dureté sera de préférence de l'ordre de 35 shore A, sans que cet exemple soit limitatif. Selon une des modalités de l'invention, chaque pliure 14 située au niveau de la circonférence de chaque disque, ainsi que chaque pliure 15 située au niveau de l'union entre deux disques voisins est plus résistante que les faces des disques, soit parcequ'elle est d'une plus grande épaisseur (non représentée) soit parcequ'elle comporte un renforcement quelconque. Ainsi, lorsqu'une nouvelle quantité de fluide est ajoutée au dispositif, chacun des disques va augmenter de volume jusqu'à prendre une forme sphérique, comme illustré sur la figure 3, sans avoir tendance à augmenter de diamètre. L'allongement maximum x permis par le dispositif est fonction du nombre et du diamètre des disques, du diamètre de la cavité axiale et de l'épaisseur de chaque face des disques. Selon une des modalités de l'invention, comme illustré par la figure 3, le dispositif est constitué de l'empilement de sept disques, dont chaque face est d'une épaisseur de 0.5 mm, d'un diamètre de 12 mm, et dont la cavité axiale est d'un diamètre de 2.5 mm. Un tel dispositif aura une capacité totale d'allongement de sept centimètres environ, chaque apport de 1 ml de fluide procurant un allongement de 1 cm environ du dispositif. Les chiffres ci-dessus sont donnés à titre d'exemple, et il est loisible de modifier un ou plusieurs de ces paramètres selon les besoins. Selon une autre modalité, dans le but d'éviter l'augmentation de diamètre et de guider l'allongement en limitant ses capacités de flexion, l'ensemble de l'accordéon peut être engainé dans une chemise non représentée, qui pourrait être analogue à la gaine 3 de la figure 1.

Les modalités de l'invention ci-dessus décrivent un système monobloc, où tous les éléments constitutifs du dispositif d'extension sont intégrés dans un ensemble, seul le détendeur de pression étant extérieur au système.

Selon une des modalités de l'invention, telle qu'illustrée par la figure 4, la chambre de pression 2, et le détendeur de pression 11 sont séparés du corps de l'implant et du dispositif extensible, et réalisent ainsi un ensemble bi-corps relié par une tubulure 17 qui transmet le fluide sous pression par un raccordement 18 à la lumière de la gaine 3, afin de refouler le corps de l'implant 7. En pareil cas, pour peu qu'elle soit du volume approprié et à la pression suffisante, la chambre de pression peut être remplie une fois pour toutes, sans nécessiter d'injections itératives. En effet, au fur et à mesure que le corps caverneux s'allonge du fait de l'expansion tissulaire, la pression interne du segment extensible baisse, et le détendeur permet alors un nouveau passage de fluide vers le segment extensible, ce qui rétablit sa pression interne initiale et entraîne une nouvelle expansion tissulaire. Celle-ci se fait donc à pression quasi-constante et de façon automatique.

Pour éviter la multiplication des figures, on n'a représenté qu'un exemple de réalisation dans la figure 4, mais les divers éléments constitutifs du système pourront être répartis différemment l'un par rapport à l'autre, et situés à un emplacement différent dans l'implant selon les besoins. Dans un tel système bi ou multi-corps, l'apport de fluide peut se faire soit par injection dans la chambre 2, soit selon une autre modalité de l'invention, par pression sur une petite pompe (non représentée) qui par un système de connexion approprié permet l'apport de fluide vers le segment extensible, ce qui permet de se passer de toute injection.

A l'inverse, selon une des modalités de l'invention non illustrée, la totalité des éléments du dispositif y compris le détendeur de pression et la pompe, peuvent être intégrés dans le corps de l'implant, ce qui permet de se passer de toute tubulure externe de raccordement.

Les applications de l'invention sont toutes les conditions où il est nécessaire obtenir un allongement important et définitif de la verge. Les indications principales sont représentées par les pathologies d'atrophie pénienne
soit congénitales, comme par exemple les micro-pénis, les micro-phallus et les epispadias.
soit acquises, comme les fibroses caverneuses de la maladie de La Peyronie.

## Revendications

1. Implant pénien pour allonger les corps caverneux de manière irréversible et définitive, par utilisation du phénomène d'expansion tissulaire, et comportant un détendeur de pression, une chambre de pression, un dispositif extensible et un corps en matériau synthétique plein, destiné à être coupé à la longueur voulue puis coiffé d'un embout.

2. Implant selon la revendication précédente, caractérisé en ce que l'embout comprend une partie sphéro-conique et une collerette, et est creusé d'une ou plusieurs cavités destinées à faire office de ventouse.

3. Implant pénien selon la revendication 1 caractérisé en ce qu'il comporte une chambre de pression de forme extérieure sphéro-conique, creusé d'une cavité dont la paroi destinée à être ponctionnée, est renforcée d'une feuille d'un matériau résistant de manière à permettre plusieurs injections trans-cutanées par une aiguille, et munie d'une valve anti-retour de manière à ce que tout passage de fluide de la chambre vers le dispositif extensible soit rendue irréversible.

4. Implant pénien selon l'une quelconque des revendications précédentes, caractérisé en ce qu'il comporte un détendeur de pression réglable, situé entre la seringue et l'aiguille de ponction de la chambre de pression.

5. Implant pénien selon l'une quelconque des revendications précédentes, caractérisé en ce que le segment extensible est constitué du corps de l'implant coulissant dans une gaine.

6. Implant pénien selon l'une quelconque des revendications précédentes, caractérisé en ce que le segment extensible est constitué d'un empilement de disques creux, communiquant entre eux par une perforation en leur centre.

7. Implant pénien, selon l'une quelconque des revendications précédentes, caractérisé en ce que le segment extensible s'allonge spontanément par la détente d'un ressort mis en place sous sa forme comprimée .

8. Implant pénien selon l'une quelconque des revendications précédentes, caractérisé en ce que la totalité des éléments qui le constituent, est intégrée dans le corps de l'implant de façon à former un ensemble monobloc.

9. Implant pénien selon l'une quelconque des revendications précédentes, caractérisé en ce qu'une ou plusieurs parties des éléments qui le constituent sont situées à distance du corps de l'implant de façon à former un ensemble bi-corps ou multi-corps.

10. Implant pénien selon l'une quelconque des revendications précédentes, caractérisé en ce que le corps de l'implant est constitué d'une matière synthétique qui pour un diamètre de 12 mm se raccourcit de l'ordre de 2 à 6% lorsqu'elle est soumise à une compression axiale de 500 g.

11. Implant pénien selon l'une quelconque des revendications précédentes, caractérisé en ce que le corps de l'implant est constitué d'une matière synthétique d'une dureté de l'ordre de 20 à 50 shore A environ.
